(19) Europäisches Patentamt
European Patent Office
Office européen des brevets

(11) Publication number: **0 120 210**
**A1**

# (12) EUROPEAN PATENT APPLICATION

(21) Application number: 84100749.5

(22) Date of filing: 25.01.84

(51) Int. Cl.³: A 61 K 7/32
A 61 K 7/34, A 61 K 7/38

(30) Priority: 28.02.83 US 470851

(43) Date of publication of application:
03.10.84 Bulletin 84/40

(84) Designated Contracting States:
BE CH DE FR GB IT LI NL SE

(71) Applicant: AMERICAN CYANAMID COMPANY
1937 West Main Street P.O. Box 60
Stamford Connecticut 06904(US)

(72) Inventor: Nabial, Wanda
172 Market Street
Garfield New Jersey(US)

(72) Inventor: Porosoff, Harold
100 Catherine Road
Scarsdale New York(US)

(72) Inventor: Miskewitz, Regina
79H Farm Road
Somerville New Jersey 08876(US)

(72) Inventor: Barbuscio, Frank
130 Andover Drive
Wayne New Jersey 07470(US)

(74) Representative: Wächtershäuser, Günter, Dr.
Tal 29
D-8000 München 2(DE)

## (54) Method for the preparation of wax-like antiperspirant compositions having improved physical characteristics.

(57) Method for preparing a wax-like antiperspirant stick product which is highly coherent and resists crumbling, and has for the principal base components a higher fatty alcohol and a volatile silicone, which comprises adding from about 0.06 to 2 percent by weight of an ingredient, which modifies the crystal formation in the base components. Such added ingredient may be, for example, eicosyl alcohol or stearic acid. The antiperspirant stick as prepared by the method.

EP 0 120 210 A1

METHOD FOR THE PREPARATION OF WAX-LIKE ANTIPERSPIRANT COMPOSITIONS HAVING IMPROVED PHYSICAL CHARACTERISTICS

The present invention relates to an improved method for preparing solid wax-like antiperspirant compositions containing an antiperspirant astringent compound and to the product prepared thereby which has a more coherent structure and less tendency to crumble.

Antiperspirant sticks, wherein the antiperspirant astringent compound is suspended or dispersed in a low melting, water-insoluble waxy material, and mixtures of waxy materials with both water-soluble and water-insoluble emollients, are known; see, for example, U.S. Patent 4,049,792. More recently, antiperspirants of the "dry feeling" type have been offered. These usually comprise a suspension of the astringent compound in a hydrophobic vehicle, such as a volatile silicone, or an emulsion of an aqueous solution of an astringent in a similar vehicle as the continuous phase.

Davy et al., U. S. Patent 4,126,679, and Geary et al., U.S. Patent 4,151,272, describe antiperspirant stick compositions comprising a suspension of an astringent in a waxy matrix containing a waxy material and volatile silicone.

In the preparation of solid stick type antiperspirants, the solid waxy ingredients are usually heated to a molten state and combined with any fluid or pulverluent materials, the molten mixture is poured into molds, which may be the package in which the product is sold, and the molten material is allowed to cool and solidify to form the antiperspirant stick.

We have found that the cooled sticks may have a crystalline structure, wherein the crystals are large, needle-like crystals in a dendritic pattern, i.e., the crystals are oriented perpendicular to the outer surface of the stick. This causes the stick to weaken along the lines of crystal orientation so that it is friable and crumbles easily under pressure or jarring.

It is therefore an object to produce a solid stick antiperspirant which has a coherent physical consistency and does not readily crumble.

Another object of this invention is to provide an antiperspirant stick in which any crystals are small and are randomly oriented.

A further object is to provide a process for producing a solid stick antiperspirant, with a non-crumbling structure of small, randomly oriented crystals.

These and other objects will become apparent herein as follows.

Essentially, the compositions of the invention comprise an active astringent compound suspended in a waxy matrix system containing the emollients described hereinabove.

Among the useful astringents are aluminum chlorhydrate, aluminum chloride, aluminum sulfate, aluminum sulfocarbolate, aluminum zirconium chlorhydrate,zinc sulfate, zirconium salts, such as zirconium chlorhydrate, combinations of aluminum chloride and aluminum-zirconium chlorhydrate, aluminum-zirconium chlorhydroglycine, and the like. Aluminum chlorhydrate and aluminum-zirconium chlorhydrate are the preferred astringents and most preferably with an average particle size of about 40-50 microns, but the particle size may be as low as 10 microns. The amount of active astringent may vary from about 15 to 35 percent by weight, preferably about 20-30 percent by weight.

The waxy matrix for the antiperspirant sticks comprises from about 15 to 25 percent by weight of stearyl alcohol. The waxy base has been found to be too soft if significantly less than about 15% stearyl alcohol is used. Stearyl alcohol, which is the preferred low melting waxy base for the compositions of the invention, may be replaced in

whole or in part by other low melting $C_{15}$ to $C_{22}$ alcohols, that is, melting between about 100-175°F, such as behenyl, cetyl, alcohol, palmitic acid, behenic acid, arachidonic acid, paraffins and the like.

An emulsifier, such as polyoxyethylene (100) stearyl alcohol, may be added to improve the stick matrix and to facilitate removal of the antiperspirant during bathing. A number of other surfactants may be suitable for this purpose, for example, sorbitan fatty esters, polyethylene sorbitol lanolin derivatives, polyoxyethylene lanolin derivatives, polyoxyethylene fatty ethers, polyethylene (25) propylene glycol stearate, polyoxyethylene stearates, polyoxyethylene sorbitan fatty ethers, and the like. Preferred are highly ethoxylated fatty alcohols, that is, containing 100-200 moles of combined ethylene oxide consisting of ethoxylated stearyl or cetyl alcohols or stearates. The emulsifier is present about 1-3%.

The emollient system of the invention comprises about 15 to 65 percent by weight of a volatile silicone. About 15 to 25 percent by weight of a liquid polyoxypropylene-alkyl ether, such as polyoxypropylene (14) butyl ether, and up to 5 percent by weight of mineral oil or other similar oils such as castor oil. The latter component preferably comprises about 1 to 3 percent by weight. The silicone/polyoxypropylene-alkyl ether ratio is preferably about 1:1, with both ingredients being present in an amount of about 20% by weight.

Volatile silicones are nonstaining and non-sticky. Silicones volatile under normal conditions of use which include certain cyclic silicones, that are oligomers of dimethyl siloxane, are preferred. Examples of such cyclic silicones include the methyl tetramer 2,4,6,9-octamethylcyclotetrasiloxane, the cyclic pentamer 2,4,6,8,10-decamethylcyclopentasiloxane and the cyclic hexamer 2,4,6,8,-10,12-dodecamethylcyclohexasiloxane. The pentamer is preferred.

The cyclic silicones are isolated from the hydrolysis product of dimethyldichlorosilane; see Patnode et al.,

J. Am. Chem. Soc. 68, 358(1946).

Polyoxypropylene-alkyl ethers useful in the invention are liquid members of the products of condensation of propylene oxide with an aliphatic alcohol represented by the formula:

$$RO\text{—}(\text{ CH—}CH_2\text{—O—})_n\text{—H}$$

A preferred number is polyoxypropylene (14)-butyl ether.

Other emollients which can be used for practical replacement of silicone include fatty acid esters (for example), isopropyl myristate, isopropyl palmitate), branched chain fatty acid esters (for example, 2-ethylhexyl palmitate), polyoxyalkylene glycol esters (for example, polypropyleneglycol 2000-monoleate), propyleneglycol diesters of short chain dicarboxylic acids (for example, $C_8$ to $C_{10}$ dicarboxylic acids), polyethylene ethers (for example, polyoxyethylene (4) lauryl ether), polyoxyethylene fatty acids, higher fatty alcohols (for example, oleyl, hexadecyl, lauryl).

In the process for producing wax-like antiperspirant sticks, the solid or wax-like ingredients are charged to a heated reactor and melted at, for example, about 65°C. The antiperspirant active material and any suspending or oily materials are then added under agitation. Finally, any fragrance, color or preservative agents are added. The molten mixture is then passed to a holding tank kept at a temperature of 50 to 60°C.

Stick packages are continuously moved on a conveyor belt and filled from the holding tank. The molten mixture in the stick packages is then allowed to cool and solidify, which cooling may be aided by passing the packages through cooling tunnels.

As previously discussed, when the sticks are cooled, the waxy matrix may crystallize in long, needle-like crystals which are oriented perpendicular to the outer surface of the stick. This causes planes of weakness along the lines of orientation of the crystals with the result that the sticks are friable and crumble easily under pressure or shock.

It has now been found that the inclusion of a small amount, 0.06-2%, preferably 0.06-1.5% by weight, of a crystal modifying material prevents the consequent dendritic arrangement of the crystallized ingredients on cooling of the molten mixture. Some suitable materials, for example, have been found to be higher fatty materials and bentone powder. Higher fatty compounds such as eicosyl alochol ($C_{20}$) or stearic acid ($C_{18}$) are suitable for the invention.

While we do not wish to be bound by the following explanation, it appears that the eicosyl alcohol acts to form seed crystals at a temperature slightly above the temperature at which the main mass of the stick would crystallize. This prevents formation of the long needle-like crystals of the matrix in a dendritic pattern, and instead causes smaller crystals to form and which will be randomly oriented. The result is a more coherent matrix. Thus what is necessary, and this is the point of the present invention, is the addition of a "seed material" which promotes the formation of crystals and thus the formation of more but smaller crystals, randomly oriented. As previously mentioned, higher hydrocarbon materials containing 18 to 20 carbon atoms have been found to be suitable. Thus a stick is formed which has a coherent physical consistency and does not crumble with usage. The amount of seed material added is important since below about 0.06 percent, the desired effect is not obtained. If the amount of seed material is greater than about 2 percent, the antiperspirant stick will not have the desired consistency, and may be too soft or too hard, depending on the added material.

The following specific Examples will serve to illustrate the application.

Example 1

An antiperspirant stick of the following compositions was prepared.

| | Percent by Weight |
|---|---|
| Aluminum chlorhydrate (44 microns) | 27.5 |
| Cyclic silicone pentamer | 26.89 |
| Stearyl alcohol | 20.00 |
| PPG(14) butyl ether | 19.20 |
| Mineral Oil | 2.0 |
| POE-100 Stearyl alcohol | 1.5 |
| Color, fragrance | 0.81 |
| | 100.00 |

Stearyl alcohol, ethoxylated stearyl alcohol, and the cyclic silicone are melted together at 65°C. and kept agitated. The aluminum chlorohydrate is added at 65°C. and stirred until homogeneously dispersed. Finally, the fragrance and color are added. The batch is then allowed to cool to 50 to 60°C., poured into suitable containers and then cooled to 40°C.

The resulting sticks obtained had a long needle--like crystalline structure, the needles being oriented perpendicular to the outer surface of the stick, and were crumbly.

When 0.7 percent of eicosyl alcohol was added to the formulation, sticks obtained from formulation cooled to 50-60°C. had a structure with randomly oriented small crystals and did not crumble.

The same result was obtained with one percent, 1.5 percent and 2 percent eicosyl alcohol.

When less than 0.06 percent eicosyl alcohol was added, sticks obtained from formulation cooled to 50-60°C. were crystalline in structure, with long needle-like crystals oriented in a dendritic pattern perpendicular to the outer surface of the stick.

When 2.5 percent eicosyl alcohol was added, the sticks obtained were too hard in consistency.

The following additional stick formulations were prepared according to the invention.

Example 2

| | Percent by Weight |
|---|---|
| Aluminum chlorhydrate (10 to 44 microns) | 25.0 |
| Stearyl alcohol | 20.00 |
| Cyclic silicone pentamer | 53.00 |
| Stearic Acid | 0.50 |
| POE-100 stearyl alcohol | 1.00 |
| Color, fragrance | 0.50 |
| | 100.00 |

Example 3

| | Percent by Weight |
|---|---|
| Volatile Silicone | 54.65 |
| Stearyl Alcohol | 15.00 |
| $C_{20}$ Alcohol | 1.00 |
| Castorwax MP-8 | 3.00 |
| Talc | 2.00 |
| PEG-8-Distearate | 1.50 |
| Propylparaben | 0.15 |
| Cab-O-Sil | 1.50 |
| Al Zr Chlorhydrate | 20.00 |
| $TiO_2$ | 0.20 |
| Fragrance and color | 1.00 |
| | 100.00 |

Examples 4 and 5

| | Percent by Weight | |
|---|---|---|
| | 4 | 5 |
| Volatile Silicone | 55.35 | 55.43 |
| Stearyl Alcohol | 15.00 | 15.00 |
| $C_{20}$ Alcohol | 0.50 | 1.00 |
| Castorwax | 3.00 | 3.00 |
| Butylated Hydroxy toluene | -- | 0.02 |
| Talc | 2.00 | 2.00 |
| PEG-8-Distearate | 1.50 | 1.50 |
| Propylparaben | 0.15 | 0.15 |
| Cab-O-Sil | 1.50 | 1.50 |
| Al Zr Chlorhydrate | 20.00 | 20.00 |
| $TiO_2$ | 0.20 | 0.20 |
| Fragrance and color | 0.80 | 0.20 |
| | 100.00 | 100.00 |

Example 6

| | Percent by Weight |
|---|---|
| Volatile Silicone | 55.55 |
| Stearyl Alcohol | 15.00 |
| $C_{20}$ Alcohol | 0.10 |
| Castorwax | 3.00 |
| Talc | 2.00 |
| PEG-8-Distearate | 1.50 |
| Propylparaben | 0.15 |
| Cab-O-Sil | 1.50 |
| Al Zr Chlorhydrate | 20.00 |
| $TiO_2$ | 0.20 |
| Fragrance & Color | 1.00 |
| | 100.00 |

Example 7

| | Percent by Weight |
|---|---|
| Stearalkoniumhectorite (Bentone 27) | 0.50 |
| Propylene carbonate | 0.15 |
| PPG-14-butyl ether | 19.00 |
| POE 100 Stearyl ether | 1.50 |
| Volatile Silicone | 26.00 |
| Mineral Oil | 2.00 |
| Stearyl alcohol | 20.00 |
| Stearic acid | 1.00 |
| 12-hydroxy stearic acid | 1.00 |
| Aluminum chlorhydrate | 27.50 |
| Fragrance and color | 1.35 |
| | 100.00 |

Examples 2 to 7 have essentially the same properties as Example 1.

WE CLAIM:

1. A wax-like antiperspirant stick having a structure of randomly oriented small crystals, said stick comprising an antiperspirant active material, a wax-like base material, an emollient, and from 0.06 to 2 percent by weight of a crystal modifying seed material.

2. The antiperspirant stick of Claim 1 wherein the seed material is a higher fatty hydrocarbon material having from 18 to 20 carbon atoms.

3. The antiperspirant stick of Claim 1 wherein the higher fatty hydrocarbon material is eicosyl alcohol or stearic acid.

4. The antiperspirant stick of Claim 1 wherein the amount of higher fatty hydrocarbon material is from 0.06 to 2.0 percent by weight.

5. The antiperspirant stick of Claim 1 wherein said antiperspirant active compound comprises an astringent metal salt.

6. The antiperspirant stick of Claim 5 wherein said antiperspirant active compound is aluminum chlorhydrate.

7. The antiperspirant stick of Claim 5 wherein said antiperspirant active compound is aluminum zirconium tetrachlorhydrex glycine.

8. The antiperspirant stick of Claim 1 comprising a volatile silicone and stearyl alcohol.

9. The antiperspirant stick of Claim 1 wherein said seed material is a non-reactive powdery solid.

10. The antiperspirant stick of Claim 9 wherein said powdery material is bentone powder.

European Patent Office

# EUROPEAN SEARCH REPORT

0120210
Application number

EP 84100749.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | US - A - 4 229 432 (NAVIN GERIA)<br>* Claims 1-5,8; examples 2,4-6 *<br>-- | 1,2, 4-6,8, 9 | A 61 K 7/32<br>A 61 K 7/34<br>A 61 K 7/38 |
| D,X | US - A - 4 151 272 (D.C. GEARY et al.)<br>* Claims 1,2,8,9,11; examples 2,5 *<br>-- | 1,2, 4-6,8, 9,10 | |
| D,X | US - A - 4 126 679 (P.F. DAVY et al.)<br>* Claims 1,2,5,7,8,13,15; example VI *<br>-- | 1,2, 5-8 | |
| D,A | US - A - 4 049 792 (W.H. ELSMAU)<br>* Claims 1-5 *<br>-- | 1,2, 4-7,9 | TECHNICAL FIELDS SEARCHED (Int. Cl. 3) |
| A | US - A - 4 083 956 (D.L. SHELTON)<br>* Claims 1-3,6,8-10; examples 1-3 *<br>-- | 1,2, 5-7,9, 10 | A 61 K 7/00<br>A 61 K 9/00 |
| A | DE - A1 - 3 003 493 (DOW CORNING LTD.)<br>* Example 4 *<br>-- | 1-3,5, 6 | |
| A | DD - B - 12 008 (B. ZDRALEK)<br>* Totality *<br>---- | 1-3 | |

The present search report has been drawn up for all claims

| Place of search | Date of completion of the search | Examiner |
|---|---|---|
| VIENNA | 01-06-1984 | MAZZUCCO |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82